# EUROPEAN PATENT SPECIFICATION

(11) **EP 3 041 508 B1**
(45) Date of publication and mention of the grant of the patent: **14.02.2018**
(21) Application number: 14759310.7
(22) Date of filing: 11.07.2014
(51) Int. Cl.: A61K 41/00, A61K 9/10, A61K 9/00, A61K 9/50, A61K 49/00, A61K 47/69, A61P 35/00, A61P 31/00

(54) **A NANOSTRUCTURE FOR THE VEHICULATION OF GAS AND/OR ACTIVE INGREDIENTS AND/OR CONTRAST AGENTS AND USES THEREOF**
NANOSTRUKTUR ZUR BEFÖRDERUNG VON GAS UND/ODER WIRKSTOFFEN UND/ODER KONTRASTMITTELN UND VERWENDUNGEN DAVON
NANOSTRUCTURE DESTINÉE À TRANSPORTER DES GAZ ET/OU DES PRINCIPES ACTIFS ET/OU PRODUITS DE CONTRASTE ET UTILISATIONS ASSOCIÉES

(30) Priority: 02.09.2013 IT TO20130707
(43) Date of publication of application: 13.07.2016
(73) Proprietor: Università Degli Studi Di Torino, 10124 Torino (IT)
(72) Inventor: CAVALLI, Roberta, 15121 Alessandria (IT); GUIOT, Caterina, 10064 Pinerolo (Torino) (IT); PRATO, Mauro, 10139 Torino (IT); TROIA, Adriano, 10137 Torino (IT)
(74) Representative: Comoglio, Elena
(86) International application number: PCT/IB2014/063025
(87) International publication number: WO 2015/028901

(56) References cited:
- SABRINA CAPECE ET AL: "A general strategy for obtaining biodegradable polymer shelled microbubbles as theranostic devices", CHEMICAL COMMUNICATIONS, vol. 49, no. 51, 1 January 2013 (2013-01-01), page 5763, XP055109628, ISSN: 1359-7345, DOI: 10.1039/c3cc42037j
- CAVALLI R ET AL: "Ultrasound-mediated oxygen delivery from chitosan nanobubbles", INTERNATIONAL JOURNAL OF PHARMACEUTICS, ELSEVIER BV, NL, vol. 378, no. 1-2, 13 August 2009 (2009-08-13), pages 215-217, XP026348620, ISSN: 0378-5173 [retrieved on 2009-06-06]
- KAWABATA K ET AL: "Phase-shift nanoparticle system for ultrasonic imaging and therapy", ULTRASONICS SYMPOSIUM, 2005 IEEE ROTTERDAM, THE NETHERLANDS 18-21 SEPT. 2005, PISCATAWAY, NJ, USA,IEEE, vol. 1, 18 September 2005 (2005-09-18), pages 637-640, XP010898821, DOI: 10.1109/ULTSYM.2005.1602933 ISBN: 978-0-7803-9382-0

## Description

Sabrina Capece et al., Chem. Commune., 2013, 49, 5763, discloses microvesicles comprising a decafluoropentane core and a polymer shell as theranostic devices. The present invention is related to nanostructures for the vehiculation of gases and/or active ingredients and/or contrast agents characterized in that they comprise a liquid decafluoropentane core and a shell made of natural or synthetic polymeric material. Such nanostructures are employed for the treatment of superficial tissue hypoxias as well as other pathologies or, in the case of contrast agent vehiculation, for the diagnosis of infectious or inflammatory or tumor diseases. In the following description the nanostructure including a liquid core and a polymeric shell may be alternatively named as a "nanodroplet".

In the context of the present invention, a nanodroplet is a nanostructure with size lower than 1 µm (1000 nm) at room temperature.

The nanodroplet has preferably a mean diameter comprised between 200 and 850 nm, more preferably comprised between 485 and 745 nm, even more preferably comprised between 350 and 750 nm, that is to say a diameter comparable to the dimensions of the pores that are present or are inducible by ultrasound-triggered sonophoresis, within dermal epithelia and with the microvascular window. The nanodroplets with size lower than 1 µm are able to penetrate through the epithelium reaching a hypoxic tissue region and acting selectively on that region.

The core of the nanodroplet of the present invention is constituted by decafluoropentane, which is a fluorocarbon molecule characterized by 5 carbon atoms linked to 10 fluorine atoms and 2 hydrogen atoms, with a molecular mass of 152,05 dalton and a boiling point of 51°C at room temperature. At human body temperature (37°C) decafluoropentane is liquid.

The shell of the nanodroplet is advantageously constituted by a natural polymer such as for example chitosan, dextran, hyaluronic acid polymers, pullulan, agarose. Alternatively, the shell of the nanodroplet can be constituted by a synthetic polymer such as for example polyethylene, polypropylene, polylactic acid.

Preferred examples are nanodroplets with a decafluoropentane core and a shell constituted by a natural polymer selected from the group comprising chitosan, dextran, pullulan, agarose, hyaluronic polyacid, and derivatives thereof.

Chitosan is a polysaccharide of natural origin obtained via partial deacetylation of chitin, retrievable in shellfish. It can also be present in some microorganisms and fungi, such as yeast. The term "chitosan" refers to a series of chitosan polymers with different molecular weight (from 50 kDa up to 2000 kDa), different viscosity, and different deacetylation grade (from 40% to 98%).

Dextran is characterized by a sequence of D-glucose molecules linked to each other via a α-1,6 glycosidic bond. They result from digestion of amylopectin, an accumulation form of starch in plants. In patients with high anti-dextran specific antibody titre, some allergic reactions may occur, which can be severe, even reaching anaphylactic shock. Therefore, antibody titration is deemed necessary before using such preparations.

Preferably, the nanodroplet comprises oxygen, alone or in combination with other gases, or it comprises an active ingredient, preferably a drug or a hormone or a vitamin or a siRNA, or it comprises a contrast agent suitable for use for diagnostic purposes, preferably a fluorescent dye emitting in the visible or in the close infrared or in the infrared spectrum.

Advantageously, the nanodroplets of the present invention are biocompatible, biodegradable, have mucoadhesive properties, can be sterilized and are able to enhance the dissolution velocity of gases and/or active ingredients and/or contrast agents incorporated within them.

In a preferred embodiment, the decafluoropentane nanodroplets are advantageously employed to vehiculate oxygen into hypoxic tissues. Hypoxia is related to a number of pathological conditions, from inflammation to cancer lesions to necrotic tissues induced by trauma, surgery or radiation.

The efficacy of treatment of these pathologies is related to an optimal oxygenation of hypoxic tissues. The nanodroplets are an effective tool for the vehiculation of oxygen to hypoxic tissues.

Hypoxic tissues are associated with many pathologies, including tumors, necrosis, chronic wounds, decompression accidents, arterial decompression sickness (either iatrogenic or barotraumatic), *Clostridium*-induced gas gangrene, acute and chronic infection of soft tissues due to various etiology, gangrene cutaneous ulcers in diabetic patients, carbon monoxide intoxication, crush lesions and compartment syndrome, at risk fractures, skin grafts, and flaps, multidrug-resistant chronic osteomyelitis, arterial, venous, and post-traumatic insufficiency cutaneous ulcers, post-actinic tissue lesions, sudden hypoacusis, aseptic osteonecrosis, retinitis pigmentosa, Ménière's syndrome, algodystrophy syndrome, periodontopathy, atherosclerosis, bacterial, fungal, and viral infections, simple and complicated malaria.

Preferably, the gas is oxygen alone or in combination with other gases.

In a preferred embodiment, the nanodroplet comprises oxygen and an active ingredient, preferably a drug, intended to treat possible concomitant inflammatory and/or infectious and/or tumor pathologies.

Preferably, such active ingredients may be incorporated into the nanodroplet or inserted into the thickness of the polymeric shell or covalently bonded to the shell surface.

In some embodiments, the nanodroplets may also include dyes, preferably fluorescent or absorbing in the close infrared or in the infrared spectra, suitable for use for diagnostic purposes (for instance to detect *in vitro* cellular endocytosis through confocal microscopy or to measure *in vivo* the levels of oxyhemoglobin and deoxyhemoglobin through photoacoustic analysis).

In a further embodiment, it is possible to achieve local targeting of the nanodroplets (for instance into the tumor site) by conjugating the polymer making up the shell with folic acid, whose receptors are largely expressed in tumor tissues.

The nanodroplets of the present invention can be prepared through different known techniques, such as for example complex coacervation, oil/water emulsion, sonication, solvent vaporization or precipitation.

Preferably, precipitation is performed in a homogenizer and with proper stabilizing agents, such as NaOH, esters of sorbitan and/or cholesteryl hemisuccinate. Sodium hydroxide can be alternatively replaced by reticulating agents such as for example glutaraldehyde or β-glycerophosphate.

Finally, the present invention provides nanodroplets for use in a method for the vehiculation of a gas, for example oxygen or oxygen in combination with other gases, to hypoxic tissues, comprising the step of treating nanodroplets according to the invention with ultrasounds.

Preferably hypoxic tissues are superficial.

Advantageously, the nanodroplets comprising oxygen alone or in combination with other gases and possibly a drug or an active ingredient may be administered by sonoporation or sonophoresis. They allow to vehiculate gases and possible drugs.

Ultrasounds act by causing transmembrane sonophoresis, eventually rupturing polymeric shell and consequently releasing oxygen and the drug or active ingredient into the pericellular liquid.

Advantageously, the nanodroplets comprising an active ingredient may also vehiculate active ingredients characterized by low permeability through biological membranes.

After release into blood circulation, the nanodroplet remnants, being non-toxic and biocompatible, will be biodegraded enzymatically or phagocytosed by macrophages and expelled via exocytosis-related mechanisms.

Further characteristics of the present invention will result from the following description of some examples.

### Example 1

### Preparation of liquid or gel formulations of decafluoropentane comprising oxygen and a chitosan or dextran shell.

1,5 ml 2H,3H-decafluoropentane, 0,5 ml polyvinylpyrrolidone, and 1,8 ml of ethanol solution containing 1% w/v Epikuron® 200 and 0.3% W/v palmitic acid are added in a three-neck flask containing 30 ml deionized H₂O. The aqueous solution is homogenized through a commercially available homogenizer for 2 min at 24000 revolutions per minute. The solution is then saturated with O₂ for 2 minutes up to reaching an O₂ concentration of 35 mg/L. Finally, 1,8 ml of solution containing chitosan (obtained by solving 7 g of chitosan in 193 ml acetate buffer at pH 5.5) or dextran (obtained by solving 7 g of dextran in 193 ml acetate buffer at pH 5.5) are added dropwise while the mixture is homogenized again for 2 minutes at 13000 revolutions per minute. At this stage the obtained nanodroplet aqueous formulation can: a) be stored at 4°C; b) be employed to make a nanodroplet gel formulation; c) be directly employed for research studies or therapeutic purposes. To prepare the gel formulation, 0,8 mg hydroxyethylcellulose are dissolved in 20 ml H₂O; thereafter, the obtained gel is mixed with 20 ml of the nanodroplet aqueous formulation prepared previously. Preferably, the gel formulation should be reconstituted immediately before being employed.

### Example 2

### Characterization of morphology, diameter, and charge of decafluoropentane nanodroplets comprising O₂ and shelled with chitosan or dextran.

Nanodroplet morphology is analyzed by optical microscopy, which allows to visually inspect the structure (gaseous core and polymeric membrane), the shape and the stability of the nanodroplets. Nanodroplets diameter and zeta potential are measured through photon correlation spectroscopy; diameter is obtained by calculating the inverse Laplace transform known as CONTIN and developed by Steven Provencher; zeta potential is obtained by using the Smuluchowski equation.

Decafluoropentane nanodroplets, produced according to the method described in Example 1, either with chitosan or dextran shell, have spherical shape. Chitosan nanodroplets have average dimensions comprised between 725 and 745 nm (mean diameter ± standard deviation: 726,55 ± 123,07); dextran nanodroplets have average dimensions included between 485 and 600 nm (mean diameter ± standard deviation: 596,35 ± 194,09). Chitosan nanodroplets have positive charge (mean zeta potential ± standard deviation: +35,38 ± 1 mV); dextran nanodroplets have negative charge (mean zeta potential ± standard deviation: -25,69 ± 1 mV).

### Example 3

### Determination of oxygen filled decafluoropentane nanodroplet ability to release O₂ in vitro by diffusion and comparison of release efficacy with respect to oxygen-filled nanobubbles with gaseous perfluoropentane core, O₂-saturated solution, and air-containing nanobubbles.

The concentration of O₂ released by aqueous formulations of oxygen containing decafluoropentane nanodroplets with chitosan or dextran shell is monitored up to 350 minutes through an oxymeter and compared to that released by aqueous formulations of: chitosan- or dextran-shelled perfluoropentane nanobubbles; O₂-saturated solution; and air-filled perfluoropentane chitosan- or dextran-shelled nanobubbles. The oxymeter has an accuracy of 0.01 mg/L. Before every measurement, the oxymeter is calibrated in air, waiting for reaching temperature and humidity stabilization. Results show that all O₂-containing liquid decafluoropentane nanodroplets of the present invention, either in gel or in water formulation, either dextran- or chitosan-shelled, release continuously high O₂ concentrations (16-22 mg/ml) for all the observational period (350 minutes). O₂ release is higher when oxygen containing decafluoropentane nanodroplets are employed (22 mg/ml O₂) with respect to oxygen containing gaseous perfluoropentane nanobubbles (16 mg/ml O₂). O₂-saturated solution releases immediately high O₂ levels (18 mg/ml) but such a release is not sustained over time, and decreases significantly after a few minutes from the start of monitoring. Air-containing chitosan- or dextran-shelled nanobubbles do not release significant O₂ amounts.

### Example 4

### Determination of oxygen containing liquid decafluoropentane nanodroplet ability to release O₂ through biological membranes in vitro in the presence of ultrasound and comparison of release efficacy with respect to oxygen-filled nanobubbles with gaseous perfluoropentane core and O₂-saturated solution.

To study ultrasound effects on O₂ release from nanodroplets through biological membranes, a home-made apparatus is used, constituted by two cylindrical chambers separated by a layer of pig ear skin and containing the first the liquid decafluoropentane nanodroplet solution in which oxygen is dissolved (lower chamber, coupled with a ultrasound probe with a high frequency transducer, 2.5 MHz and a power of 5 W) and the other a 0.9% NaCl hypoxic solution (upper chamber, coupled with an oxymeter to measure oxygen release).

The concentration of O₂ released from aqueous or gel formulations of liquid decafluoropentane nanodroplets with chitosan or dextran shell containing oxygen is monitored up to 135 minutes and compared with that released from aqueous or gel formulations of gaseous perfluoropentane nanobubbles with analogous chitosan or dextran shell containing dissolved oxygen and with that released from O₂-saturated aqueous solution. Ultrasound-induced O₂ release is higher when the present invention's liquid decafluoropentane nanodroplets filled with O₂ are employed (22 mg/ml O₂ released) with respect to gaseous perfluoropentane nanobubbles filled with O₂ (16 mg/ml O₂ released). O₂-saturated solution releases immediately O₂, but oxygen levels are not sustained over time.

### Example 5

### Determination of nanodroplets ability to release oxygen in vivo by diffusion.

Small portions of shaved skin of mice are treated with 5 mL of decafluoropentane nanodroplet with chitosan shell gel formulation. Thereafter mouse blood oxyhemoglobin levels are monitored for 10 minutes by photoacoustic spectroscopy.

Results show that decafluoropentane nanodroplets enhance significantly and in a time-sustained manner blood oxyhemoglobin levels.

### Example 6

### O₂-filled 2H,3H-decafluoropentane nanodroplet effects on viability of human and murine cell cultures.

Oxygen containing decafluoropentane nanodroplets are added to different types of cell cultures treated for 24 hours either in normoxia (20% O₂) or in hypoxia (1% O₂).

In particular, 100 µL dextran-shelled decafluoropentane nanodroplets are added per each mL of cultures of human monocytes (primary cells isolated from peripheral blood of healthy donors, 10⁶ cells/mL), human dermal microvascular endothelial cells (HMEC-1 cell line, 10⁵ cells/mL), and murine alveolar macrophages (MH-S cell line, 10⁵ cells/mL); after 24 hours, the cytotoxic effect of nanodroplets is measured by calculating the rate between the concentration of lactate dehydrogenase (LDH) released by cells into culture medium and the sum of concentrations of intracellular and extracellular LDH. LDH activity, proportional to concentration, is measured through a common spectrophotometric assay performed in the presence of piruvate and NADH. Cell viability is also measured through a spectophotometric assay based on the use of 3-(4,5-dimethylthiazol-2-yl)-2,5-diphenyltetrazolium bromide (MTT). Results from both assays show that nanodroplets are not toxic to any analyzed cultures (citotoxicity always lower than 10%) and that cells keep a normal viability grade.

Similar results are obtained by treating human keratinocytes (HaCaT cell line, 10⁵ cells/mL) with oxygen containing decafluoropentane nanodroplets with chitosan shell (also in this case 100 µL nanodroplets are added per mL of culture medium).

### Example 7

### Effect Oxygen containing decafluoropentane nanodroplet on hypoxia-dependent dysregulation of matrix metalloproteinase (MMP) and their tissue inhibitor (TIMP) secretion by human keratinocytes.

Human keratinocytes (HaCaT cell line, 10⁵ cells/mL) are cultured in parallel in normoxia (20% O₂) and in hypoxia (1% O₂) in the presence or absence of oxygen containing decafluoropentane nanodroplets with chitosan shell (100 µL per mL of culture medium). Thereafter, cell supernatants are collected and the levels of MMPs and TIMPs are measured by means of immunoenzimatic assay (ELISA). Results show that, in the absence of nanodroplets, human keratinocytes secrete constitutively MMP -2 and MMP-9, as well as their inhibitors TIMP-1 and TIMP-2, in the range of pg/mL, when they are cultured in normoxia. The culture in hypoxia, in the absence of nanodroplets, dysregulates the secretion, with MMP-2, MMP-9, and TIMP-2 secretion being reduced.

These hypoxia-dependent dysregulating effects are eliminated when oxygen containing decafluoropentane nanodroplets are added to cultures, and the physiological basal levels of secretion by cells are restored.

### Example 8

### Effect of oxygen containing decafluoropentane nanodroplet on hypoxia-dependent dysregulation of matrix metalloproteinase (MMP) and their tissue inhibitor (TIMP) secretion by human monocytes.

Human monocytes (primary cells isolated from peripheral blood of healthy donors, 10⁶ cells/mL) are cultured in parallel in normoxia (20% O₂) and in hypoxia (1% O₂) in the presence or absence of oxygen containing decafluoropentane nanodroplets with dextran-shell (100 µL per mL of culture medium). Thereafter, cell supernatants are collected and the levels of MMPs and TIMPs are measured by means of immunoenzimatic assay (ELISA). Results show that, in the absence of nanodroplets, human monocytes secrete constitutively , in the range of pg/mL, MMP-9, as well as MMP inhibitors TIMP-1 and TIMP-2 when they are cultured in normoxia. When cultured in hypoxia, in the ansence of nanodroplets, secretion is dysregulated, with TIMP-1 secretion being enhanced, and MMP-9 and TIMP-2 secretion being reduced.

These hypoxia-dependent dysregulating effects are eliminated when oxygen containing decafluoropentane nanodroplets are added to cultures, and the physiological basal levels of secretion by cells are restored.

### Example 9

### Effect of oxygen containing decafluoropentane nanodroplets with chitosan shell, either vancomycin-coniugated or not, on methicillin-resistant Staphylococcus aureus (MRSA) bacterial growth.

10⁵ CFU/mL MRSA are cultured at 37°C in tryptic soy broth in the presence or absence of oxygen containing decafluoropentane nanodroplets with chitosan shell (1 mL nanodroplets in 10 mL of bacterial culture). Thereafter bacterial growth is evaluated by counting CFU after 2, 3, 4, and 6 hours from the beginning of incubation. Results show that in the presence of nanodroplets not conjugated with vancomycin bacterial growth is reduced of 2 magnitude orders at all the monitored times. The use of vancomycin-conjugated nanodroplets completely stops bacterial growth, and kills MRSA population.

### Example 10

### Effect of oxygen containing decafluoropentane nanodroplets with chitosan shell on Candida albicans fungal growth.

10⁴ CFU/mL *Candida albicans* are cultured at 37°C in Sabouraud Dextrose Agar in the presence or absence of oxygen containing decafluoropentane nanodroplets with chitosan shell (1 mL nanodroplets in 10 mL of *Candida* culture) and fungal growth is evaluated after 2, 3, 4, 6, and 24 hours from the beginning of incubation. Results show that in the presence of nanodroplets fungal growth is reduced of 2 orders of magnitude at all the monitored times.

### Example 11

### Effect of oxygen containing decafluoropentane nanodroplets on MMP-9 secretion by human monocytes after phagocytosis of malarial pigment (hemozoin).

Human monocytes (primary cells isolated from peripheral blood of healthy donors, 10⁶ cells/mL) are treated in hypoxia for 2 hours with malarial pigment. After washings, monocytes are incubated for 24 hours in the presence or absence oxygen containing decafluoropentane nanodroplets with dextran shell (100 µL per mL of culture medium). Thereafter, cell supernatants are collected and analyzed by zymography. Results show that malarial pigment promotes a 7-fold enhancement of MMP-9 release, and such an effect is eliminated in the sample where nanodroplets have been added.

### Example 12

### Effect of oxygen containing decafluoropentane nanodroplets with dextran shell on phenotype of human monocytes treated with 15-HETE (a lipoperoxide present in the atheromatous plaque during atherosclerosis).

Human monocytes (primary cells isolated from peripheral blood of healthy donors, 10⁶ cells/mL) are treated in hypoxia for 2 hours with micromolar concentrations (1-10 µM) of 15-HETE. After washings, monocytes are incubated for 24 hours in the presence or absence of oxygen containing decafluoropentane nanodroplets with dextran shell (100 µL per mL of culture medium).

Thereafter, cell supernatants are collected and analyzed by zymography. Results show that 15-HETE promotes a 7-fold enhancement of MMP-9 release, and such an effect is eliminated in the sample where nanodroplets have been added.

## Claims

1. A nanostructure for the vehiculation of gas and/or active ingredient and/or contrast agent having a diameter comprised within the range of from 200 nanometers and less than 1000 nanometers, **characterized in that** said nanostructure comprises a liquid core of decafluoropentane and an outer shell of natural or synthetic polymeric material.

2. The nanostructure according to claim 1, wherein the polymeric material is selected from the group consisting of chitosan, dextran, hyaluronic acid, agarose and pullulan.

3. The nanostructure according to claim 1 or 2, which comprises a gas dissolved in decafluoropentane.

4. The nanostructure according to any of claims 1 to 3, wherein the gas dissolved in decafluoropentane is selected from the group consisting of oxygen (O2), nitrogen monoxide (NO) and hydrogen (H2).

5. The nanostructure according to any of claims 1 to 4, which comprises an active ingredient, preferably a drug.

6. The nanostructure according to claim 5, wherein the drug is selected from the group consisting of antibacterial, anti-mycotic, anti-malarial, antiviral, anti-inflammatory, cytotoxic, anti-metabolic, anti-tumoral and anti-atherosclerotic.

7. The nanostructure according to claim 6, wherein the drug is selected from the group consisting of vancomycin, fluconazole, chloroquine, artemisinin, artemisinin derivatives, primaquine, quinine, dexamethasone, acetylsalicylic acid, doxorubicin, epirubicin, 5-fluorouracyl, methotrexate.

8. The nanostructure according to any of claims 1 to 7, having a diameter comprised between 200 nm and 850 nm, preferably comprised between 485 nm and 745 nm.

9. The nanostructure according to any of claims 1 to 8, wherein the polymer of the polymeric material of the outer shell is conjugated with a compound selected from the group consisting of folic acid; dyes absorbing in the visible, near infrared or infrared; fluorescent dyes emitting in the visible, near-infrared or infrared.

10. The nanostructure according to any of claims 1 to 9, for use in the treatment of a disease selected from the group consisting of hypoxias, infectious diseases and tumor diseases.

11. The nanostructure for use according to claim 10, wherein the disease is selected from the group consisting of tumors, necrosis, chronic wounds, decompression accidents, iatrogenic arterial gas embolism, barotraumatic arterial gas embolism, clostridial gas gangrene, soft tissues infections, gangrene and skin ulcers from diabetes, carbon monoxide poisoning, crush injuries, compartment syndrome, fractures at risk, skin grafts and flaps at risk, chronic refractory osteomyelitis, skin ulcers from arterial insufficiency, venous insufficiency skin ulcers, post-traumatic insufficiency cutaneous ulcers, post-actinic tissue lesions, sudden hearing loss, aseptic osteonecrosis, pigmentary retinopathy, Ménière's syndrome, algodystrophy syndrome, periodontopathy, atherosclerosis, bacterial infections, fungal infections, viral infections, simple or complicated malaria.

12. The nanostructure according to any of claims 1 to 9, for use in enhancing the effects of radiotherapy in the treatment of a shallow tumor.

## Patentansprüche

1. Nanostruktur zur Beförderung von Gas und/oder Wirkstoffen und/oder Kontrastmittel mit einem Durchmesser, der innerhalb des Bereichs von 200 Nanometer und weniger als 1000 Nanometer enthalten ist, **dadurch gekennzeichnet, dass** die Nanostruktur einen Flüssigkern aus Dekafluorpentan und eine Außenhülle aus natürlichem oder synthetischem Polymermaterial enthält.

2. Nanostruktur nach Anspruch 1, wobei das Polymermaterial aus der Gruppe gewählt ist, die besteht aus: Chitosan, Dextran, Hyaluronsäure, Agarose und Pullulan.

3. Nanostruktur nach Anspruch 1 oder 2, die ein in Dekafluorpentan gelöstes Gas enthält.

4. Nanostruktur nach einem der Ansprüche 1 bis 3, wobei das in Dekafluorpentan gelöste Gas aus der Gruppe gewählt ist, die besteht aus: Sauerstoff (O2), Stickstoffmonoxid (NO) und Wasserstoff (H2).

5. Nanostruktur nach einem der Ansprüche 1 bis 4, die einen Wirkstoff, vorzugsweise ein Medikament, enthält.

6. Nanostruktur nach Anspruch 5, wobei das Medikament aus der Gruppe gewählt ist, die besteht aus: antibakteriell, anti-mykotisch, anti-malarial, antiviral, anti-inflammatorisch, zytotoxisch, anti-metabolisch, anti-tumoral und anti-atherosklerotisch.

7. Nanostruktur nach Anspruch 6, wobei das Medikament gewählt ist aus der Gruppe, die besteht aus: Vancomycin, Fluconazol, Chloroquin, Artemisinin, Artemisinin-Derivative, Primaquin, Chinin, Dexamethason, Acetylsalicylsäure, Doxorubicin, Epirubicin, 5-Fluoruracyl, Methotrexat.

8. Nanostruktur nach einem der Ansprüche 1 bis 7, die einen Durchmesser hat, der enthalten ist zwischen 200 nm und 850 nm, vorzugsweise enthalten ist zwischen 485 nm und 745 nm.

9. Nanostruktur nach einem der Ansprüche 1 bis 8, wobei das Polymer des Polymermaterials der Außenhülle mit einer Verbindung konjugiert ist, die aus der Gruppe ausgewählt ist, die besteht aus Folsäure; Farbstoffen, die im Sichtbaren, in der Nähe von Infrarot oder im Infrarotbereich absorbieren; fluoreszierenden Farbstoffen, die im Sichtbaren, in der Nähe von Infrarot oder im Infrarotbereich emittieren.

10. Nanostruktur nach einem der Ansprüche 1 bis 9 zur Verwendung in der Behandlung einer Krankheit, die ausgewählt ist aus der Gruppe, die besteht aus Sauerstoffmangel, Infektionskrankheiten und Tumorkrankheiten.

11. Nanostruktur zur Verwendung nach Anspruch 10, wobei die Krankheit gewählt ist aus der Gruppe, die besteht aus Tumoren, Nekrose, chronischen Wunden, Dekompressionsunfällen, iatrogener arterieller Gasembolie, barotraumatischer arterieller Gasembolie, clostridialer Gasgangrän, Weichgewebeinfektionen, Wundbrand und Hautgeschwüren von Diabetes, Kohlenstoffmonoxidvergiftung, Quetschverletzungen, Kompartmentsyndrom, Risikobrüchen, Hauttransplantationen und -lappen mit Risiko, chronische refraktäre Osteomyelitis, Hautgeschwüre aus arterieller Insuffizienz, venöse Insuffizienz Hautgeschwüre, posttraumatische Insuffizienz kutane Geschwüre, postaktinische Gewebeläsionen, plötzlicher Gehörverlust, aseptische Osteonekrose, Retinitis pigmentosa, Ménière's Syndrom, Algodystrophiesyndrom, Periodontopathie, Atherosklerose, bakterielle Infektionen, Pilzinfektionen, virale Infektionen, einfache oder komplizierte Malaria.

12. Nanostruktur nach einem der Ansprüche 1 bis 9, zur Verwendung bei der Verstärkung der Wirkungen von Strahlentherapie bei der Behandlung eines oberflächlichen Tumors.

## Revendications

1. Nanostructure destinée à véhiculer un gaz et/ou un principe actif et/ou un agent de contraste ayant un diamètre compris dans l'intervalle de 200 nanomètres à moins de 1000 nanomètres, **caractérisée en ce que** ladite nanostructure comprend un noyau liquide de décafluoropentane et une enveloppe externe constituée de matériau polymère naturel ou synthétique.

2. Nanostructure selon la revendication 1, dans laquelle le matériau polymère est choisi dans le groupe constitué par le chitosane, le dextrane, l'acide hyaluronique, l'agarose et le pullulane.

3. Nanostructure selon la revendication 1 ou 2, qui comprend un gaz dissous dans du décafluoropentane.

4. Nanostructure selon l'une quelconque des revendications 1 à 3, dans laquelle le gaz dissous dans le décafluoropentane est choisi dans le groupe constitué par l'oxygène (O2), le monoxyde d'azote (NO) et l'hydrogène (H2).

5. Nanostructure selon l'une quelconque des revendications 1 à 4, qui comprend un principe actif, de préférence un médicament.

6. Nanostructure selon la revendication 5, dans laquelle le médicament est choisi dans le groupe constitué par un antibactérien, un antimycotique, un antipaludéen, un antiviral, un anti-inflammatoire, un cytotoxique, un antimétabolique, un anti-tumoral et un anti-athéroscléreux.

7. Nanostructure selon la revendication 6, dans laquelle le médicament est choisi dans le groupe constitué par la vancomycine, le fluconazole, la chloroquine, l'artémisinine, les dérivés d'artémisinine, la primaquine, la quinine, la dexaméthasone, l'acide acétylsalicylique, la doxorubicine, l'épirubicine, le 5-fluorouracyle, le méthotrexate.

8. Nanostructure selon l'une quelconque des revendications 1 à 7, ayant un diamètre compris entre 200 nm et 850 nm, de préférence compris entre 485 nm et 745 nm.

9. Nanostructure selon l'une quelconque des revendications 1 à 8, dans laquelle le polymère du matériau polymère de l'enveloppe externe est conjugué à un composé choisi dans le groupe constitué par l'acide folique ; les colorants absorbant dans le visible, le proche infrarouge ou l'infrarouge ; les colorants fluorescents émettant dans le visible, le proche infrarouge ou l'infrarouge.

10. Nanostructure selon l'une quelconque des revendications 1 à 9, destinée à être utilisée dans le traitement d'une maladie choisie dans le groupe constitué par les hypoxies, les maladies infectieuses et les maladies tumorales.

11. Nanostructure destinée à être utilisée selon la revendication 10, où la maladie est choisie dans le groupe constitué par les tumeurs, les nécroses, les blessures chroniques, les accidents de décompression, l'embolie gazeuse artérielle iatrogénique, l'embolie gazeuse artérielle barotraumatique, la gangrène gazeuse clostridienne, les infections des tissus mous, la gangrène et les ulcères cutanés dus au diabète, l'empoisonnement au monoxyde de carbone, les blessures par écrasement, le syndrome du compartiment, les fractures à risque, les greffes de peau et les lambeaux de peau à risque, l'ostéomyélite réfractaire chronique, les ulcères de la peau dus à une insuffisance artérielle, les ulcères de la peau dus à une insuffisance veineuse, les ulcères cutanés dus à une insuffisance post-traumatique, les lésions tissulaires post-actiniques, la perte auditive soudaine, l'ostéonécrose aseptique, la rétinopathie pigmentaire, le syndrome de Ménière, le syndrome algodystrophique, la parodontopathie, l'athérosclérose, les infections bactériennes, les infections fongiques, les infections virales, le paludisme simple ou grave.

12. Nanostructure selon l'une quelconque des revendications 1 à 9, destinée à être utilisée pour renforcer les effets de la radiothérapie dans le traitement d'une tumeur peu profonde.
